# EUROPEAN PATENT SPECIFICATION

(11) **EP 3 930 658 B1**
(45) Date of publication and mention of the grant of the patent: **01.01.2025**
(21) Application number: 20713368.7
(22) Date of filing: 21.02.2020
(51) Int. Cl.: A01N 1/02, A61M 1/02

(54) **PORTABLE AIR BLAST SYSTEM FOR HOMOGENEOUS AND REPRODUCIBLE FREEZING AND THAWING OF BIOLOGICAL MATERIALS**
TRAGBARES DRUCKLUFTSYSTEM ZUM HOMOGENEN UND REPRODUZIERBAREN EINFRIEREN UND AUFTAUEN VON BIOLOGISCHEN MATERIALIEN
SYSTÈME DE SOUFFLAGE D'AIR PORTABLE POUR CONGÉLATION ET DÉCONGÉLATION HOMOGÈNES ET REPRODUCTIBLES DE MATÉRIAUX BIOLOGIQUES

(30) Priority: 27.02.2019 PT 2019115345
(43) Date of publication of application: 05.01.2022
(73) Proprietor: Smartfreez Lda, 2740-122 Porto Salvo (PT)
(72) Inventor: SILVESTRE DUARTE, Andreia Filipa, 6230-654 Silvares Fnd (PT); SENA REGO, Pedro Gil, 1000-225 Lisboa (PT); DE BRITO ESTRELA, Rui, 1500-714 Lisboa (PT)
(74) Representative: Patentree
(86) International application number: PCT/IB2020/051479
(87) International publication number: WO 2020/174338

(56) References cited:
- CN-U- 204 746 359
- US-A1- 2003 080 126
- US-A1- 2009 049 845

## Description

### TECHNICAL FIELD

This disclosure relates, in general, to systems and methods for homogeneous and reproducible freezing and thawing of aqueous solutions of biological materials, in particular those used in chemical, and pharmaceutical processes. This disclosure relates to a system and a method to improve the uniformity of the heat transfer coefficient of containers filled with biological materials during the freezing and thawing process, in particular a system that is easily transported and incremented to other conventional freeze-thaw methods and equipment.

### BACKGROUND

Biological materials are produced industrially in large batches that are stored for extended periods of time and are typically frozen to minimize degradation during its relatively long shelf life. The process of freeze-thaw of biological solutions poses several challenges that need to be considered, such as freeze-thaw operation parameters, storage of substance, shipping in frozen state, and logistics. The choice of the storage container is one of the most important consideration of the freeze-thaw process since they can have an impact on stability and downstream operations of a formulation. Commonly used containers for frozen biological materials include bags and bottles/carboys. Although the disposable bags offer advantages over bottles/carboys, the potential risk of breakage of a bag or tubing assemblies leading to contamination risk and loss of product is a bigger concern as compared to bottles/carboys. Bottle/carboy systems are simple and more robust than bags, and if a biological formulation is robust and stable under a wide range of freeze-thaw conditions, this is the preferred mode of operation for many companies.

Currently, the freezing process involves placing the bottles and/or carboys comprising the biological solution in conventional upright or chest freezers and allowing the product to freeze. However, product losses are often observed when biological solution freezing is carried out using conventional freezers with set-point temperatures ranging from -20 °C to -80 °C. This is attributed to the inadequacy of such freezers to provide the cooling rates necessary for a given load configuration. Conventional freezers lack uniform cooling throughout the entire freezer space since the containers are placed in the freezer side by side and sometimes stacked, resulting in different thermal gradients for different containers. Under these conditions, the freezing rate and product quality is thus dependent on freezer capacity, freezer load, space between containers, container size, container shape, and airflow properties inside the freezer. Another option of the freezing process involves the use of cold air and blast freezers to rapidly freeze the biological material below their glass transition temperature. In a typical air-blast freezer, the cooled air is circulated by fans over the containers confined in an insulated closed room or chamber. The extent of cooling achieved depends on several factors such as position of fans, chamber capacity, load configuration, temperature and velocity of airflow. Even when using this fast freezing method, freezing heterogeneity can occur from container to container; consequently, heterogeneity in the quality of a product batch may occur.

Another consequence of different freezing rates is the heterogeneity in solutes distribution (macro cryoconcentration or freeze-concentration) that occurs at a macro scale in frozen solutions of biological materials. Cryoconcentration has been associated with heterogeneous ice matrix, which could result in degradation of biological material and quality loss; further, slow freezing and thawing compromises the stability of biological substances. Furthermore, heat transfer at the top of the containers, both by convection and radiation, leads to the formation of an ice crust consisting an ice layer on top of the liquid, at the air-liquid interface. The ice crust contributes to increased internal pressure in the containers and consequently results in damage or rupture of the containers. Portuguese national provisional patent application No. 115152, filed on November 12, 2018 disclosed an insulator device to prevent ice crust formation on the top of the liquid, at the air-liquid interface, in the head-space region of containers. This device allows improvement in the freezing process by avoiding ice-crust formation and the increase in pressure inside the containers thus preventing container damage or rupture.

Although bottles and carboys have been widely used in many biopharmaceutical companies, the existence of scale-down models that mimic large-scale systems is desired for developmental studies and optimization of the freezing-thawing process. Recently, document WO2018211437A1 discloses a scale-down system designed for bottles. US2003/080126A1 is directed to a system for freezing and thawing a biopharmaceutical material in a container. CN204746 359U and US 2009/049845A1 refer to portable storage devices for containers.

Although there are already systems and methods that help to improve the process of freezing and thawing using bottles and carboys, such as the ice crust insulator device and the scale-down system, these systems still cannot solve the problem of freezing heterogeneity in multiple containers within a single batch. Specifically, the problem of freezing heterogeneity when using currently available freezers, conventional or blast freezers, and freezers being located in different sites. Most of the time, freezing equipment are already installed and differs from site to site in the same biopharmaceutical company, therefore it is desirable to design a simple, portable system for homogeneous and reproducible freezing and thawing of biological materials which can be used in conjunction with existing equipment in different locations. Moreover, it may be convenient that the system and method can incorporate the already disclosed ice crust insulator device and the scale-down system, thus preventing the above described problems. This will allow container to container homogeneity of the freezing process even when using different freezer types.

### GENERAL DESCRIPTION

The present disclosure provides a system and a method for homogeneous and reproducible freezing and thawing of biological materials, in particular, a system that is easily transported and used in conjunction with other conventional freeze-thaw methods and equipment.

This disclosure provides a system and a method to improve the uniformity of the heat transfer coefficient of containers filled with biological materials during the freezing and thawing process.

The system disclosed includes a portable air blast system configured to receive a container filled with biological materials and to be placed in a cooling or heating chamber. The system allows homogeneous and reproducible freezing and thawing of biological materials. This portable air blast system improves the uniformity of the heat transfer coefficient in the walls of the container, thus allows similar freezing/thawing rates to be achieved even when using different cooling or heating chambers.

The portable air blast system, is disclosed in claim 1.

Another aspect of this disclosure relates to a method to improve the uniformity of the heat transfer coefficient of containers filled with biological materials during the freezing and thawing process, using the presently disclosed system, comprising the steps of: (1) providing a cooling or heating chamber; (2) providing at least one portable air blast system; (3) placing the container in the center of the portable air blast system; (4) placing the portable air blast system inside the cooling or heating chamber; and (5) turning on the fan until the biological material completely freezes or thaws.

Another aspect of this disclosure relates to the use of a plurality of portable air blast systems combined in a single chamber and a trolley to quickly load the chamber with the portable air blast system, or a plurality of portable air blast systems.

Another aspect relates to a platform configured to receive the portable air blast system that can provide rotation, rocking, shaking, vibrations or other forms of mechanical motion to induce the convection of the liquid inside the container. The platform is to be used during the thawing process.

An aspect relates to a system for freezing and thawing aqueous solutions of biological materials, in particular, a system to improve the uniformity of the heat transfer coefficient of containers filled with biological materials, comprising:
a portable airblast system configured to receive a container;
a container filled with an aqueous solution of biological materials;
a cooling or heating chamber, to receive the portable air blast system, for freezing or thawing.

In an embodiment, the portable air blast system is configured to receive a container, is made of a rigid material, such as plastic, polymer or other material having high rigidity.

In an embodiment, the stand may have a support designed accordingly to receive the container.

In an embodiment, the stand may have pins to connect the stand to the vent enclosure.

In an embodiment, the pins maintain a distance between the stand and the vent enclosure ranging from 2 cm to 5 cm.

In an embodiment, the distance between the walls of the vent enclosure and the side walls of the container is approximately constant and comprised between 1 cm and 3 cm.

In an embodiment, the air velocity inside the vent should preferably be higher than double of the outside air downward velocity.

In an embodiment, the air within the vent has a velocity in a range from approximately 0.5 m/s to approximately 20 m/s, preferably in a range from approximately 1 m/s to approximately 10 m/s, and more preferably from approximately 2 m/s to approximately 8 m/s.

In an embodiment, the fan may have a support to be connected to the vent enclosure, preferentially an insulating support.

In an embodiment, the fan is located at the top of the vent enclosure, preferably above the container.

In an embodiment, a fan or a blower can be used, preferably a fan is used, more preferably an axial fan is used.

In an embodiment, the fan uses batteries.

In an embodiment, the fan may have a control system, preferentially to control velocity.

In an embodiment, the fan should be suitable for use in a cryogenic environment.

In an embodiment, more than one fan can be used, preferably the fans are juxtaposed horizontally or vertically.

In an embodiment, the portable air blast system could be made of modular segments that attach to each other.

In an embodiment, the container is a container of fixed shape.

In an embodiment, the container is made of a rigid and biocompatible material such as of glass, polyethylene terephthalates, polycarbonate, polytetrafluoroethylene, polyethylene, polyesters, polyamides, polypropylenes, ethylene-vinyl alcohol copolymer, polyvinylidene fluoride, polyvinylchlorides, and copolymers, mixtures or laminates that comprise the above.

In an embodiment, the container is a deformable container.

In an embodiment, the deformable container is made of a biocompatible polymeric material such as, ethylene-vinyl acetate copolymer, ethylene-vinyl alcohol copolymer, polytetrafluoroethylene, polyethylene, polyesters, polyamides, polypropylenes, polyvinylidenefluoride, polyurethanes, polyvinylchlorides, and copolymers, mixtures or laminates that comprise the above.

In an embodiment, the deformable container has a volumetric capacity in a range from approximately 10 mL to approximately 20 L, preferably in a range from approximately 10 mL to approximately 1 L.

In an embodiment, the deformable container is placed inside a rigid shell, preferentially made of a material with low heat resistance such as a metal (for instance, stainless steel, aluminium or copper).

In an embodiment, the container may comprise an ice-crust attenuator device.

In an embodiment, the container may comprise a scale-down device.

In an embodiment, the cooling or heating chamber may be static or be an air blast, or a controlled temperature room.

In an embodiment, a plurality of portable air blast systems could be combined in a single chamber.

In an embodiment, there is a typical the distance between the several portable air blast systems to assure that the air outside the vent has a velocity in a range from approximately 0.05 m/s to approximately 1 m/s, preferably in a range from approximately 0.1 m/s to approximately 0.5 m/s.

In an embodiment, a trolley is used to load the chamber with the portable air blast system, or a plurality of portable air blast systems.

In an embodiment, a platform configured to receive the portable air blast system can provide rotation, rocking, shaking, vibrations or other forms of mechanically inducing the convection of the liquid inside the container.

### BRIEF DESCRIPTION OF THE DRAWINGS

These and other objects, features and advantages of the disclosure will be evident from the following detailed description when read in conjunction with the accompanying drawings.

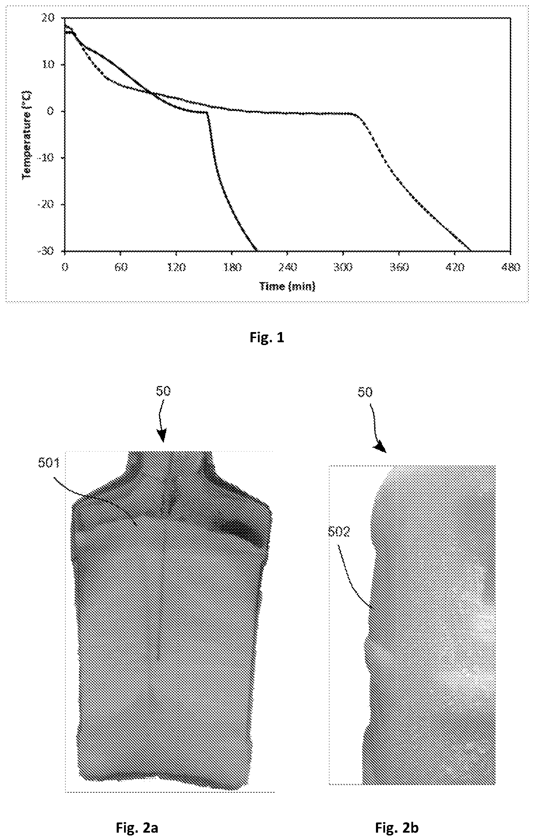

For an easier understanding of the disclosure the attached drawings are joined, which represent preferred embodiments of the disclosure that, however, are not meant to limit the object of the present application.
[0001] **Figure 1** shows the time-temperature profile inside a bottle during the freezing process when using blast freezer (solid line) or static freezer (dashed line).
**Figure 2a** is a cross-section view of a bottle **50** frozen in an air blast freezer with a lateral fan. This cross-section view shows the heterogeneity of the ice growth **501.**
**Figure 2b** is a lateral view of a bottle **50** frozen in an air blast freezer with a lateral fan. This lateral view shows the lateral deformation **502** of the bottle **50.**
**Figure 3** is a front view of a portable air blast system **10** configured according to the present disclosure.
**Figure 4** is a schematic cross-section view of a portable air blast system **10** with a container **50** and an ice crust attenuator device **60.** The air blast system is configured according to the present disclosure.
**Figure 5** is a perspective exploded view of a portable air blast system **10** comprising a stand **20,** a vent enclosure **30** and a fan enclosure **40.** The air blast system is configured according to in the present disclosure.
**Figure 6** is a partial exploded and elevated view of a portable air blast system **10** configured according to the present disclosure.
**Figure 7** is an exploded schematic cross-section view of a portable air blast system **10** with a container 50 and an ice crust attenuator device **60.** The air blast system is configured according to the present disclosure.
**Figure 8** is an exploded schematic cross-section view of a portable air blast system **10** with a deformable container **70** inside a rigid shell **701.** The air blast system is configured according to the present disclosure.
**Figure 9** shows the time-temperature profile inside a bottle during the freezing process in a static freezer using (solid line) or not using (dashed line) a portable air blast system **10.**
**Figure 10a** is a cross-section view of a bottle **50** frozen in static freezer using a portable air blast system **10.** The air blast system is configured according to the present disclosure. The cross-section view shows a planar ice surface **504.**
**Figure 10b** is a cross-section view of a bottle **50** frozen in static freezer, showing the ice crust formation and the "pyramidal" shape **501.**
**Figure 11** is a perspective view of multiple portable air blast systems **10** placed in a cooling or heating chamber **80.** The air blast system is configured according to the present disclosure.
**Figure 12** is an exploded section view of a portable air blast system 10 designed in a modular assembly, configured according to the present disclosure.

### DETAILED DESCRIPTION

As described above, the process of freezing and-thawing of biological materials poses several challenges; the lack of homogeneity associated with the freezing and thawing phenomena is one of the main problems. Many variables contribute to freezing inconsistency, the major issue relates to the different freezing rates experienced by multiple containers within a single batch of product. Currently, the freezing process involves placing the bottles and/or carboys comprising the biological materials in a conventional upright or chest freezers, or blast freezers and then allowing the product to freeze. The freezing rate and product quality is dependent on the freezer capacity, freezer load configuration, space between containers, container size, container shape, and airflow properties inside the freezer. When using blast freezers, although the freezing time is faster than using a static freezer **(****Figure 1****),** freezing heterogeneity occurs and this is mainly due to the properties of the airflow (direction and velocity). Depending on the load and configuration of the freezer, this heterogeneity might be higher. In addition to the heterogeneity in the freezing rates between containers, a very important aspect is the heterogeneity of the ice growth **501** in the container **50 (****Figure 2a****).** This heterogeneity in freezing rates leads to the problem of cryoconcentration and possibly also deformation **502** or rupture of the container **50 (****Figure 2b****)** due to uncontrolled growth of ice and increased internal pressure. Therefore, it is crucial to have a system and method to freeze and thaw biological materials in a homogeneous and reproducible manner, ensuring controlled ice growth and avoiding the internal pressure build-up.

Another problem relates to the existence of different freezing and thawing equipment used in several sites of the same biopharmaceutical company. As such, it is desirable to have a portable system, simple for use in different locations, in order to achieve homogeneous and reproducible freezing and thawing of biological materials and can be used in conjunction with existing equipment. Moreover, having a portable system enables the shipping of the air blast system together with any product thus allowing accurate thawing in another location which may have lesser technical capabilities. This ensures the quality of the product and reproducibility of the process throughout the supply chain even when using different equipment.

The present disclosure describes a system and method that enables improvement in the uniformity of the heat transfer coefficient of the external surface of containers filled with biological materials during the freezing and thawing process, while preventing damage or rupture of the containers. The portable system disclosed allows any chamber to be used for freezing or thawing, and also significantly decreases the time required to freeze or thaw a biological material in a container.

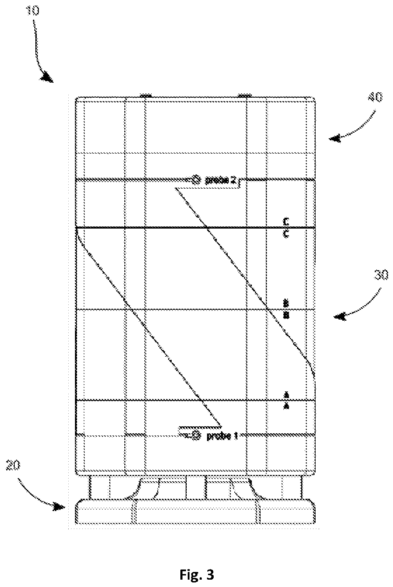

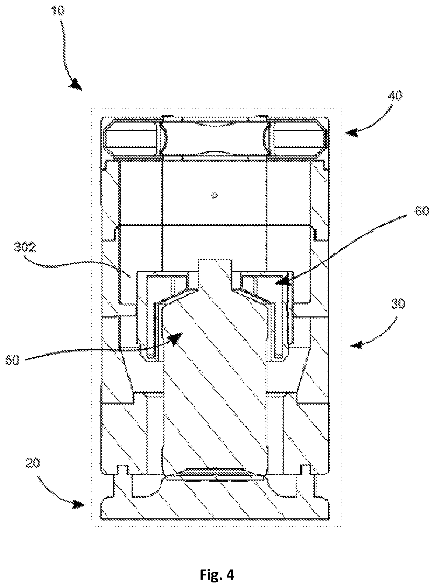

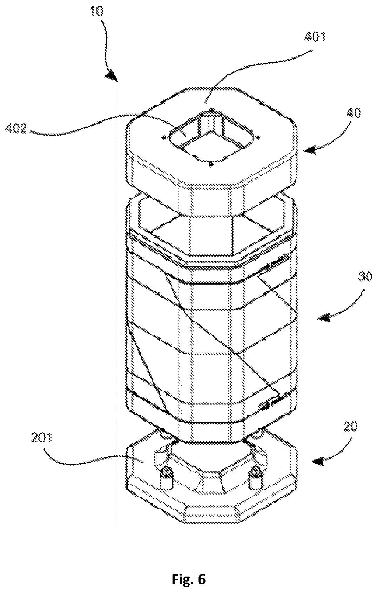

In an embodiment, the system is configured to receive a container filled with biological materials for freezing and thawing. The system includes a portable air blast system **10** configured to receive a container **50** filled with biological materials. Said portable air blast system **10** is configured to be placed in a cooling or heating chamber, for homogeneous and reproducible freezing and thawing of biological materials, respectively. The main purpose of said portable air blast system is to improve the uniformity of the heat transfer coefficient in the walls of the container, achieving similar freezing/thawing rates even using different cooling or heating chambers. (See Figures 3 to 8 for example illustration)

The portable air blast system **10** comprises a stand **20,** a vent enclosure **30** and a fan enclosure **40.** (See Figures 3 to 8 for illustrations) The portable air blast system is made of a rigid material, such as plastic, polymer, or other material with high rigidity.

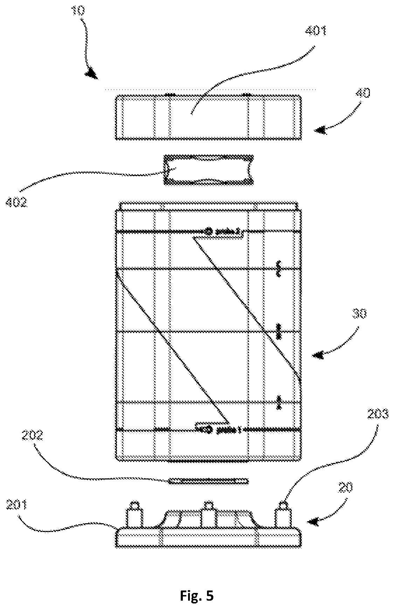

The stand **20** is designed to receive a container **50** and to maintain the container's position in the center of the vent enclosure **30,** in order to achieve a uniform distribution of air on all the side walls of the container. The stand **20** may have several supports **202** to receive the container **50,** designed accordingly to the container to be used with. Said stand **20** may have a solid base **201** and pins **203** to connect the stand **20** to the vent enclosure **30** and to maintain a distance between the stand **20** and the vent enclosure **30.** The distance between the stand **20** and the vent enclosure **30** ranges from 1 cm to 10 cm, preferably ranging from 2 cm to 5 cm, this is to ensure that air is distributed into the vent **302.** When the portable air blast system **10** is placed inside an air blast chamber with lateral ventilation, the stand **20** may have an opening below, to ensure that air is uniformly distributed in the vent **302.** In another embodiment, the container **50** can be maintained in the center of the vent enclosure **30** by a support claw connected to the vent enclosure **30.** (See Figure 5 for illustration)

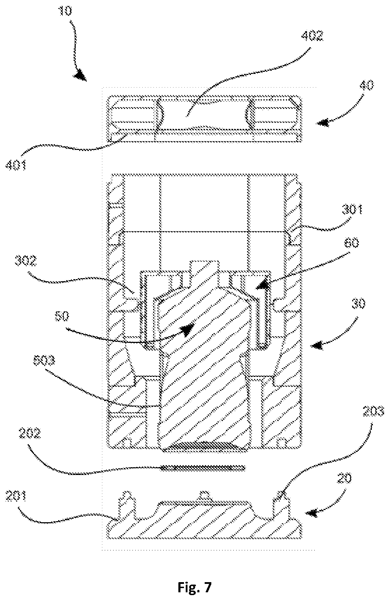

The portable air blast system **10** comprises a vent enclosure **30** designed to create a vent **302,** in order to obtain a vertical velocity field that is substantially identical around all side walls **503** of the container. To ensure similar vertical velocity field around all side walls **503** of the container, the distance between the walls of the vent enclosure **301** and the side walls **503** of the container is substantially constant and comprised between 1 cm and 10 cm, preferably between 1 cm and 3 cm. The vent enclosure **30** may decouple the vertical airflow that passes inside the vent **302** of the downward flow that passes outside. The air velocity inside the vent **302** should preferably be higher than the double of the outside air downward velocity. In a preferred embodiment, the air within the vent **302** has a velocity ranging from approximately 0.5 m/s to approximately 20 m/s, preferably ranging from approximately 1 m/s to approximately 10 m/s, and more preferably from approximately 2 m/s to approximately 8 m/s. (See Figure 7 for illustration)

The portable air blast system **10** comprises a fan enclosure **40** with a fan **402** to force air in the chamber or room to pass through the vent **302** in order to improve the uniformity of the heat transfer coefficient on the container's walls during the freezing and thawing process. In a preferred embodiment, the fan **402** is located at the top of the vent enclosure **30,** above the container, by using an insulating support **401.** In an embodiment, a fan or a blower is used, preferably a fan **402** is used, more preferably an axial fan is used. In another embodiment, batteries could be used to power on the fan, and can be packed inside the insulating support **401.** In another embodiment, the velocity of the fan **402** may also be controlled to conveniently increase or decrease the heat transfer for different stages of the process, for sensitive biological materials or for scale-down purposes. In an embodiment, said fan **402** is suitable for use in a cryogenic environment. In another embodiment, more than one fan **402** can be used to force the air to pass through the vent **302,** for example 2 or 4 fans juxtaposed horizontally or vertically, or one at the top and another at the bottom of the vent enclosure. (See Figures 5 to 7 for illustration)

In an embodiment, a temperature probe is located at one or more points within the portable air blast system and inside the container. The temperature probe provides an indication of the temperature of the airflow at a particular location inside the portable air blast system and indicates the time-temperature profile of the freezing/thawing of the biological material inside the container. Temperature probe may comprise a thermocouple, a thermistor, or other conventional temperature sensing devices suitable for use in a cryogenic environment.

In another embodiment, an air velocity probe is located at one or more points of the portable air blast system, to provide information about the airflow velocity inside the portable air blast system at a particular location. Air velocity probe comprises an anemometer, pitot tube, or other conventional sensing devices suitable for use in a cryogenic environment.

The container **50** configured to be filled with biological is a bag, a bottle or a carboy. Preferably, said container **50** should maintain its shape when empty and do not significantly deform when filled with product. Said container **50** can be made of a rigid and biocompatible material to promote compatibility with biological materials. The materials can be, for instance, glass, polyethylene terephthalates, polycarbonate, polytetrafluoroethylene, polyethylene, polyesters, polyamides, polypropylenes, ethylene-vinyl alcohol copolymer, polyvinylidenefluoride, polyvinylchlorides, copolymers, and mixtures or laminates that comprise the abovementioned. Said container **50** may vary in size and volumetric The container **50** has a volumetric capacity ranging from approximately 1 mL to approximately 20L. Said container **50** configured to be filled with biological materials may comprise a head-space region, and one cap with at least one port with tubing for aseptic filling and venting purposes.

In an embodiment, the container **50** may also comprise an ice crust attenuator device **60** configured for attachment to the head-space of the container. The main purpose of the ice crust attenuator **60** device is to prevent the formation of the ice crust that leads to increased pressure inside the containers, and consequently resulting in their damage.

In another embodiment, the container may also comprise a scale-down device that mimic large-scale containers for development studies and optimization of the freezing-thawing process. In this embodiment using the scale-down device, the air velocity in the vent may be conveniently adjusted to control the average heat transfer coefficient, for example to match that of the large-scale container.

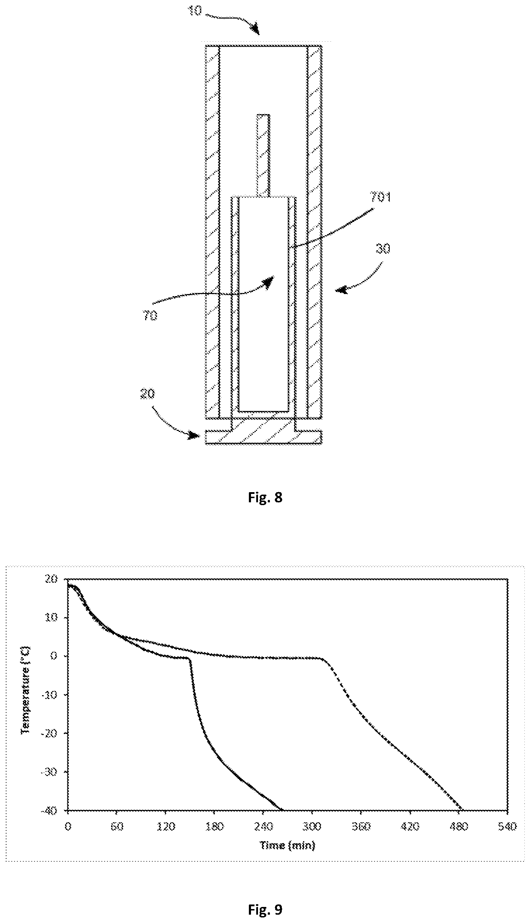

In another embodiment, deformable containers **70** may also be frozen or thawed with the portable air blast system **10,** in this case by placing the deformable container **70** inside a rigid shell **701.** The rigid shell is preferably made of a material with low heat resistance such as a metal (for instance, stainless steel, aluminum or copper). Said deformable container **70,** such as bags, may deform when filled with product, and can be made of a biocompatible polymeric material to promote compatibility with biological materials. The biocompatible polymeric materials may be, for instance, ethylene-vinyl acetate copolymer, ethylene-vinyl alcohol copolymer, polytetrafluoroethylene, polyethylene, polyesters, polyamides, polypropylenes, polyvinylidenefluoride, polyurethanes, polyvinylchlorides, and copolymers, mixtures or laminates that comprise the above. The deformable container **70,** may vary in size and volumetric capacity. In a preferred embodiment, the deformable container **70** has a volumetric capacity ranging from approximately 10 mL to approximately 20 L, preferably ranging from approximately 10 mL to approximately 1 L. (See Figure 8 for illustration)

In the present disclosure, biological materials may comprise protein, amino acid and peptide formulations, DNA, RNA and nucleic acid solutions, cell suspensions, tissue suspensions, cell aggregates suspensions, cell growth media, serum, biologicals, blood products, preservation solutions, fermentation broths, and cell culture fluids with and without cells, mixtures of the above and their fragments.

The portable air blast system **10,** depicted in **Figures 3 to 7****,** has particular relevance in a common freezing or thawing process when placed directly in a cavity of a chamber. Said chamber may or may not have convection and may be cooled or heated in order to freeze or thaw. Through the use of the fan in the portable air blast system, the air from the cooled or heated chamber will be directed parallel to the walls of the container, without interference from the other adjacent containers, ensuring that the freezing-thawing of each container is not influenced by the others. The system herein disclosed allows any chamber to be used for freezing or thawing, taking advantage of pre-existing cooled/heated air in the chamber. Moreover, the system herein disclosed is designed to be portable, it is lightweight and compact, preferably weighing less than 20 kg or by not increasing the weight and size of the filled container by more than 100%. This shipping between sites can be done easily and can be used in any chamber or room thus ensuring homogeneous, reproducible and faster freezing and thawing of biological materials.

Further examples are discussed in detail below with regard to the use of the disclosed portable air blast system to freeze an aqueous solution in a container.

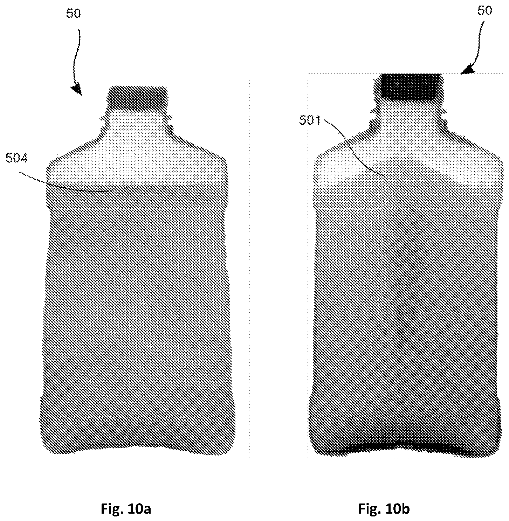

In an embodiment, for example, the portable air blast system **10** was used to freeze a volume of 1.8 L of a 5% (m/V) sucrose aqueous solution in a Polyethylene terephthalate (PET) bottle of 240 (h) x 120 (w) x 120 (d) mm of dimensions. The test was performed with an ice crust attenuator device **60** as described above. In one experiment, the bottle was placed directly inside an ultra-low freezing chamber with the temperature setpoint at -80°C and the bottle was allowed to freeze. In another experiment, the bottle was frozen inside the portable air blast system and placed in an ultra-low freezing chamber with the temperature setpoint of -80 °C. **Figure 9** illustrates the time-temperature profiles inside the bottle in both experiments, with and without the portable air blast system **10.** It can be observed that when using the portable air blast system **10,** the freezing process is faster **(****Figure 9****,** solid line). It takes about 200 min to achieve the temperature of -30 °C inside the bottle with the use of the portable air blast system, but it took about 435 min to reach the same temperature without the portable air blast system **(****Figure 9****,** dashed line). Moreover, as shown in **Figure 10****,** the ice crust formation and the "pyramidal" shape **501** on the top of the liquid are avoid when using the portable air blast system and the ice crust attenuator device, leading to a planar ice surface **504.** Therefore, these results show that the system disclosed can improve the freezing process, decreasing the freezing time by improving the heat transfer coefficient on the container's walls during the freezing process while avoiding ice crust formation **(****Figure 10a****),** consequently preventing damage of the containers.

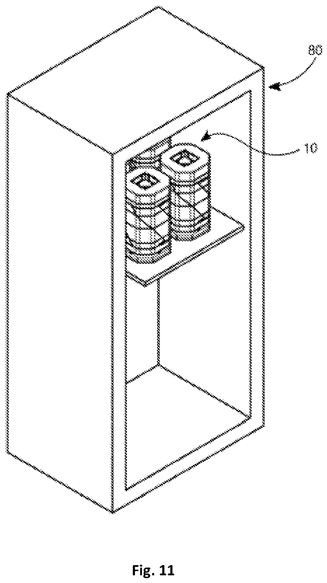

In another embodiment, a plurality of portable air blast systems **10** could be combined in a single chamber **80.** (See Figure 11 for illustration) In a preferred embodiment, each container has a portable air blast system with one fan above the container. In a preferred embodiment, there is a distance between the several portable air blast systems to ensure that the air outside the vent has a velocity ranging from approximately 0.05 m/s to approximately 1 m/s, preferably ranging from approximately 0.1 m/s to approximately 0.5 m/s.

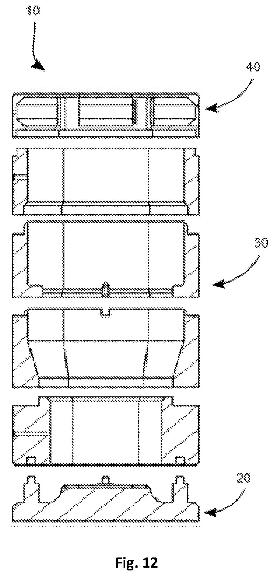

In another embodiment, the portable air blast system **10** is be made of modular segments attachable to each other. The portable air blast system **10** comprises the possibility of interchangeable modules for different functions, to receive different containers and devices. The modular assembly of the portable air blast system **10** opens up the possibility of transforming and adapting the system to different scenarios. Different modules can be added or changed to increase the dimensions of the system, to conveniently adapt to different containers, to change the air flow profile and to receive different devices such as probes, batteries, electronics, etc. The modular assembly also allows for ease of transportation, manufacturing, parts replacement and in place assembly. (See Figure 12 for illustration)

In another embodiment, the system also includes a trolley to allow quick loading of the chamber with a portable air blast system, or a plurality of portable air blast systems.

In another embodiment, the container is agitated during thawing to mix the biological solution. This can be achieved by using a platform configured to receive the portable air blast system, that can provide rotation, rocking, shaking, vibrations or other forms of mechanical motion to induce the convection of the liquid inside the container.

Another aspect of this disclosure relates to a method to improve the uniformity of the heat transfer coefficient of containers filled with biological materials during the freezing and thawing process, using the previously described system, comprising the steps of: (1) providing a cooling or heating chamber; (2) providing at least one portable air blast system; (3) placing the container in the center of the portable air blast system; (4) placing the portable air blast system inside the cooling or heating chamber; and (5) turning on the fan until the biological material completely freezes or thaws.

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the claims.

Thus, unless otherwise stated the steps described are so unordered meaning that, when possible, the steps can be performed in any convenient or desirable order.

The following claims further set out particular embodiments of the disclosure.

## Claims

1. A portable air blast system configured for freezing and thawing biological solution inside a container comprising
a vent enclosure **30** with walls **301** configured to create a vent **302** in the portable air blast system;
a stand **20** comprising several supports **202;**
wherein the stand **20** and the supports **202** are configured to receive a container with side walls **503** so that the distance between the stand and the vent enclosure **30** ranges from 1 cm to 10 cm and the position of the container is maintained in the center of the vent enclosure **30** such that the distance between the walls of the vent enclosure **301** and side walls of the container **503** ranges from 1 cm to 10 cm and is approximately constant to ensure similar vertical airflow velocity around all side walls of the container;
at least one fan, wherein the fan forces air to pass through the vent **302** for uniform heat transfer coefficient in the walls of the container **503;** a controller for controlling the vertical airflow velocity; and
a fan enclosure;
wherein the vertical airflow velocity ranges from approximately 0.5 m/s to approximately 20 m/s;
wherein the container **503** is a bag, bottle, or carboy;
and wherein the system is configured to receive a container **503** with a volumetric capacity ranging from approximately 1 mL to approximately 20 L.

2. The portable air blast system according to the previous claim, wherein the stand further comprises a solid base and pins for connecting the stand to the vent enclosure.

3. The portable air blast system according to any of the previous claims, wherein the distance between the stand and the vent enclosure ranges from 2 cm to 5 cm.

4. The portable air blast system according to any of the previous claims, wherein the distance between the walls of the vent enclosure and the side walls of the container ranges from 1 cm to 3 cm and is approximately constant to ensure a similar vertical airflow velocity around all side walls of the container.

5. The portable air blast system according to any of the previous claims, wherein the vertical airflow velocity ranges from approximately 1 m/s to approximately 10 m/s, preferably from approximately 2 m/s to approximately 8 m/s.

6. The portable air blast system according to any of the previous claims, wherein the system is made of a rigid material, preferably plastic, more preferably polymer.

7. The portable air blast system according to any of the previous claims, wherein the fan is positioned within the fan enclosure.

8. The portable air blast system according to any of the previous claims, wherein the fan is an axial fan.

9. A method of freezing and thawing biological solution inside a container by using the portable air blast system according to any of the previous claims 1 - 8, comprising:
obtaining a container filled with biological solution;
placing the container into the vent enclosure and on the stand of the air blast system,
wherein the position of the container is maintained in the center of the vent enclosure;
placing the air blast system with the container into a heating or cooling chamber;
activating the air blast system by activating the fan in the air blast system.

10. The method of freezing and thawing biological solution inside a container according to the previous claim, further comprising placing more than one air blast system into a chamber.

11. The method of freezing and thawing biological solution inside a container according to the previous claim 10, wherein each air blast system is placed at a distance from each other to ensure that the air outside the air blast system vent has a velocity ranging from 0.05 m/s to 1 m/s, preferably from 0.1 m/s to 0.5 m/s.

## Patentansprüche

1. Ein tragbares Druckluftsystem, das zum Einfrieren und Auftauen einer biologischen Lösung in einem Behälter konfiguriert ist, umfassend
ein Lüftungsgehäuse **30** mit Wänden **301,** die so konfiguriert sind, dass sie eine Entlüftung **302** in dem tragbaren Druckluftsystem bilden;
einen Ständer 20, umfassend mehrere Träger **202;**
wobei der Ständer **20** und die Träger **202** so konfiguriert sind, dass sie einen Behälter mit Seitenwänden **503** aufnehmen, so dass der Abstand zwischen dem Ständer und dem Lüftungsgehäuse **30** zwischen 1 cm bis 10 cm liegt und die Position des Behälters in der Mitte des Lüftungsgehäuses **30** beibehalten wird, so dass der Abstand zwischen den Wänden des Lüftungsgehäuses **301** und den Seitenwänden des Behälters **503** zwischen 1 cm bis 10 cm liegt und annähernd konstant ist, um eine ähnliche Geschwindigkeit des vertikalen Luftstroms um alle Seitenwände des Behälters herum sicherzustellen;
mindestens ein Gebläse, wobei das Gebläse die Luft durch die Entlüftung **302** bläst, um einen gleichmäßigen Wärmeübergangskoeffizienten in den Wänden des Behälters **503** zu erreichen; eine Steuereinheit zur Steuerung der Geschwindigkeit des vertikalen Luftstroms; und
ein Gebläsegehäuse;
wobei die Geschwindigkeit des vertikalen Luftstroms zwischen annähernd 0,5 m/s und annähernd 20 m/s liegt;
wobei der Behälter **503** ein Beutel, eine Flasche oder eine Ballonflasche ist;
und wobei das System so konfiguriert ist, dass es einen Behälter **503** mit einem Fassungsvermögen von annähernd 1 ml bis annähernd 20 l aufnehmen kann.

2. Das tragbare Druckluftsystem nach dem vorangehenden Anspruch, wobei der Ständer ferner eine feste Basis und Stifte zur Verbindung des Ständers mit dem Lüftungsgehäuse umfasst.

3. Das tragbare Druckluftsystem nach einem der vorangehenden Ansprüche, wobei der Abstand zwischen dem Ständer und dem Lüftungsgehäuse zwischen 2 cm und 5 cm liegt.

4. Das tragbare Druckluftsystem nach einem der vorangehenden Ansprüche, wobei der Abstand zwischen den Wänden des Lüftungsgehäuses und den Seitenwänden des Behälters zwischen 1 cm bis 3 cm liegt und annähernd konstant ist, um eine ähnliche Geschwindigkeit des vertikalen Luftstroms um alle Seitenwände des Behälters herum sicherzustellen.

5. Das tragbare Druckluftsystem nach einem der vorangehenden Ansprüche, wobei die Geschwindigkeit des vertikalen Luftstroms zwischen annähernd 1 m/s bis annähernd 10 m/s liegt, bevorzugt zwischen annähernd 2 m/s bis annähernd 8 m/s.

6. Das tragbare Druckluftsystem nach einem der vorangehenden Ansprüche, wobei das System aus einem steifen Material, bevorzugt Kunststoff, besonders bevorzugt Polymer, hergestellt ist.

7. Das tragbare Druckluftsystem nach einem der vorangehenden Ansprüche, wobei das Gebläse innerhalb des Gebläsegehäuses angeordnet ist.

8. Das tragbare Druckluftsystem nach einem der vorangehenden Ansprüche, wobei das Gebläse ein Axialgebläse ist.

9. Ein Verfahren zum Einfrieren und Auftauen einer biologischen Lösung in einem Behälter unter Verwendung des tragbaren Druckluftsystems nach einem der vorangehenden Ansprüche 1-8, umfassend:
Erhalten eines mit einer biologischen Lösung gefüllten Behälters;
Einsetzen des Behälters in das Lüftungsgehäuse und auf den Ständer des Druckluftsystems, wobei die Position des Behälters in der Mitte des Lüftungsgehäuses beibehalten wird;
Einsetzen des Druckluftsystems mit dem Behälter in eine Heiz- oder Kühlkammer;
Einschalten des Druckluftsystems durch Einschalten des Gebläses in dem Druckluftsystem.

10. Das Verfahren zum Einfrieren und Auftauen einer biologischen Lösung in einem Behälter nach dem vorangehenden Anspruch, ferner umfassend das Einbringen von mehr als einem Druckluftsystem in eine Kammer.

11. Das Verfahren zum Einfrieren und Auftauen einer biologischen Lösung in einem Behälter nach dem vorangehenden Anspruch 10, wobei jedes Druckluftsystem mit einem Abstand zueinander angeordnet ist, um sicherzustellen, dass die Luft außerhalb der Entlüftung des Druckluftsystems eine Geschwindigkeit von 0,05 m/s bis 1 m/s hat, bevorzugt von 0,1 m/s bis 0,5 m/s.

## Revendications

1. Un système de soufflage d'air portable configuré pour la congélation et décongélation d'une solution biologique à l'intérieur d'un conteneur comprenant
une enceinte à évent **30** avec des parois **301** configurée pour créer un évent **302** dans le système de soufflage d'air portable ;
un socle **20** comprenant plusieurs supports **202** ;
dans lequel le socle **20** et les supports **202** sont configurés pour recevoir un conteneur avec des parois latérales **503** tels que la distance entre le socle et l'enceinte à évent **30** varie entre 1 cm et 10 cm et la position du conteneur est maintenue au centre de l'enceinte à évent **30** telle que la distance entre les parois de l'enceinte à évent **301** et les parois latérales du conteneur **503** varie entre 1 cm et 10 cm et soit approximativement constante pour garantir une vitesse d'écoulement d'air vertical similaire autour de toutes les parois latérales du conteneur ;
au moins un ventilateur, dans lequel le ventilateur force l'air à passer à travers l'évent **302** pour un coefficient de transfert de chaleur uniforme dans les parois du conteneur **503 ;** un contrôleur pour contrôler la vitesse d'écoulement d'air vertical ; et
une enceinte à ventilateur ;
dans lequel la vitesse d'écoulement d'air vertical varie entre environ 0,5 m/s et environ 20 m/s ;
dans lequel le conteneur **503** est un sac, une bouteille ou une tourie ;
et dans lequel le système est configuré pour recevoir un conteneur **503** avec une capacité volumétrique variant entre environ 1 mL et environ 20 L.

2. Le système de soufflage d'air portable selon la revendication précédente, dans lequel le socle comprend également une base solide et des tiges pour connecter le socle à l'enceinte d'évent.

3. Le système de soufflage d'air portable selon l'une quelconque des revendications précédentes, dans lequel la distance entre le socle et l'enceinte à évent varie entre 2 cm et 5 cm.

4. Le système de soufflage d'air portable selon l'une quelconque des revendications précédentes, dans lequel la distance entre les parois de l'enceinte à évent et les parois latérales du conteneur varie entre 1 cm et 3 cm et est approximativement constante pour garantir une vitesse d'écoulement d'air vertical similaire autour de toutes les parois latérales du conteneur.

5. Le système de soufflage d'air portable selon l'une quelconque des revendications précédentes, dans lequel la vitesse d'écoulement d'air vertical varie entre environ 1 m/s et environ 10 m/s, préférablement entre environ 2 m/s et environ 8 m/s.

6. Le système de soufflage d'air portable selon l'une quelconque des revendications précédentes, dans lequel le système est fait d'un matériau rigide, préférablement de plastique, plus préférablement polymère.

7. Le système de soufflage d'air portable selon l'une quelconque des revendications précédentes, dans lequel le ventilateur est placé dans l'enceinte à ventilateur.

8. Le système de soufflage d'air portable selon l'une quelconque des revendications précédentes, dans lequel le ventilateur est un ventilateur axial.

9. Un procédé de congélation et décongélation d'une solution biologique à l'intérieur d'un conteneur en utilisant le système de soufflage d'air portable selon l'une quelconque des revendications précédentes 1-8, comprenant :
obtenir un conteneur rempli d'une solution biologique ;
placer le conteneur dans l'enceinte à évent et sur le socle du système de soufflage d'air, dans lequel la position du conteneur est maintenue au centre de l'enceinte à évent ;
placer le système de soufflage d'air avec le conteneur dans une chambre de chauffe ou de refroidissement ;
activer le système de soufflage d'air en activant le ventilateur dans le système de soufflage d'air.

10. Le procédé de congélation et décongélation d'une solution biologique à l'intérieur d'un conteneur selon la revendication précédente, comprenant également placer plus d'un système de soufflage d'air dans une chambre.

11. Le procédé de congélation et décongélation d'une solution biologique à l'intérieur d'un conteneur selon la revendication précédente 10, dans lequel chaque système de soufflage d'air est placé à une distance l'un de l'autre pour garantir que l'air a l'extérieur de l'évent du système de soufflage d'air a une vitesse variant entre 0,05 m/s et 1 m/s, préférablement entre 0,1 m/s et 0,5 m/s.
